# EUROPÄISCHE PATENTSCHRIFT

(11) **EP 1 274 367 B1**
(45) Veröffentlichungstag und Bekanntmachung des Hinweises auf die Patenterteilung: **05.12.2007**
(21) Anmeldenummer: 01938092.2
(22) Anmeldetag: 06.04.2001
(51) Int. Cl.: A61C 8/00, A61B 17/68, A61F 2/30, A61F 2/02

(54) **IMPLANTAT**
IMPLANT
IMPLANT

(30) Priorität: 19.04.2000 DE 10019339
(43) Veröffentlichungstag der Anmeldung: 15.01.2003
(73) Patentinhaber: Heraeus Kulzer GmbH, 63450 Hanau (DE)
(72) Erfinder: AUGTHUN, Michael, 45470 Mülheim (DE); PETERS, Manfred, 38302 Wolfenbüttel (DE); HASELHUHN, Klaus, 52064 Aachen (DE); SPIEKERMANN, Hubertus, 42718 Haan (DE)
(74) Vertreter: Kühn, Hans-Christian
(86) Internationale Anmeldenummer: PCT/EP2001/003949
(87) Internationale Veröffentlichungsnummer: WO 2001/080769

(56) Entgegenhaltungen:
- DE-A- 3 300 764
- US-A- 5 197 881
- US-A- 5 961 328

## Beschreibung

Die Erfindung betrifft ein Implantat zur Aufnahme eines Verbindungszapfens eines medizinischen Elements, mit einer Längsachse, einem distalen Ende und einem proximalen Ende, von dem aus sich eine Ausnahmeausnehmung für den Verbindungszapfen in Richtung der Längsachse in das Innere des Implantats erstreckt, wobei das Implantat an seiner äußeren Mantelfläche kraft- oder formschlüssig mit der inneren Mantelfläche einer Aufnahmebohrung in einem Knochen eines menschlichen oder tierischen Körpers verbindbar ist und wobei der an die Aufnahmeausnehmung angepaßte Verbindungszapfen kraft- oder formschlüssig mit der inneren Mantelfläche der Aufnahmeausnehmung verbindbar ist.

Derartige Implantatsysteme sind beispielsweise im zahnmedizinischen Bereich weit verbreitet. In diesem Fall wird das aufzunehmende medizinische Element, zum Beispiel von einem Ersatzzahn, einer Verschlußkappe, einem Abdruckpfosten oder einem Gingiva-Former gebildet. Der aus der WO 99/29255 bekannte Ersatzzahn besteht aus einem Verbindungszapfen mit einem Unterteil, einem Zentralteil und einem Oberteil sowie einer auf das Oberteil aufgeschobenen Krone. Der Ersatzzahn wird als Ganzes mit seinem konischen Unterteil in eine daran angepaßte, ebenfalls konische Aufnahmebohrung in einem Implantat, das zuvor in den Kieferknochen eingesetzt und darin ausreichend eingeheilt sein muß, eingesetzt. Bei der Verbindung zwischen dem Verbindungszapfen und der Aufnahmeausnehmung kommt das Prinzip eines Klemmkonus zur Anwendung. Die Möglichkeit einer Verdrehung des Ersatzzahns um die Längsachse des Verbindungszapfens erlaubt es zwar, die Winkelstellung des Ersatzzahns in bezug auf eine Rotation um seine Hochachse beim Vorgang des Einsetzens exakt anzupassen, führt jedoch zu dem Nachteil, daß insbesondere bei einer größeren Belastung eine hinreichende Verdrehsicherheit des Ersatzzahns im Implantat nicht gewährleistet ist.
Alternativ zu dem vorgenannten Implantatsystem ist eine Schraubverbindung zwischen dem Ersatzzahn und dem Implantat die am weitesten verbreitete Konnektierungsart, die sich durch ihre einfache Reversibilität auszeichnet. Als Nachteil ist jedoch hier der große Zeitaufwand für das Eindrehen der Befestigungsschrauben - insbesondere bei einer größeren Anzahl von Ersatzzähnen - anzusehen. Oftmals ist nämlich nur eine temporäre Verbindung zwischen dem medizinischen Element und dem Implantat erforderlich, so daß Erstgenanntes nach einer gewissen Zeit ohnehin wieder aus dem Implantat entfernt werden muß. Auch bei dem Vorgang des Entfernens ist im Falle von Schraubverbindungen ein zeitaufwendiges Herausdrehen der Schrauben notwendig.

Außerdem ist insbesondere bei der temporären Verbindung medizinischer Elemente mit dem Implantat eine geringere Festigkeit der Verbindung ausreichend als dies beim endgültigen Einsetzen, beispielsweise eines Ersatzzahns, der Fall ist. Eine Differenzierung der Verbindungssysteme einerseits nur temporär in das Implantat eingesetzter Elemente und andererseits dauerhaft darin verbleibender ist daher wünschenswert.
Dokumente DE 33 00 764 und US 5197881 offenbaren jeweils ein Implantat gemäß dem Oberbegriff des Anspruchs 1.
Der Erfindung liegt die Aufgabe zugrunde, ein Implantat zur Aufnahme eines Verbindungszapfens eines medizinischen Elements vorzuschlagen, bei dem sich der Verbindungszapfen auf einfache und nur wenig Zeit beanspruchende Weise mit der Aufnahmeausnehmung des Implantats verbinden läßt. Des weiteren soll die vorgenannte Verbindung gleichfalls sehr einfach und schnell wieder aufhebbar sein.

Ausgehend von einem Implantat der eingangs beschriebenen Art wird diese Aufgabe erfindungsgemäß dadurch gelöst, daß die innere Mantelfläche der Aufnahmeausnehmung mit mindestens einer sich senkrecht zur Längsachse des Implantats erstreckenden und eine Hinterchneidung bildenden Vertiefung versehen ist, mit der ein radial nach außen vorstehender Vorsprung eines elastischen Clipelements des Verbindungszapfens in Eingriff bringbar ist.

Bei dem erfindungsgemäßen Implantat läßt sich die Verbindung zwischen dem medizinischen Element und dem Implantat auf einfachste Weise durch ein Einstecken des Verbindungszapfens des Elements herstellen. Bei diesem Einstecken kommt es automatisch zum Eingreifen des Vorsprungs oder der Vorsprünge des mindestens einen Clipelements, wodurch ein Formschluß entsteht und durch die Federspannung des Clipelements eine radiale Kraft auftritt, die - bedingt durch die nach unten gerichtete Wölbung der Vertiefung im Implantat - den Verbindungszapfen axial nach unten zieht bis das Oberteil des Zapfens vollständig auf der Stirnfläche des Implantats aufliegt. Gleichzeitig wird der rückwärts gerichteten Bewegung des Verbindungszapfens aus der Aufnahmeausnehmung heraus ein Widerstand entgegensetzt. Dieser Widerstand läßt sich je nach der Steifigkeit des Clipelements sowie der Form und der Tiefe der Hinterschneidung an die jeweiligen Bedürfnisse anpassen. Wird eine der Einsatzrichtung entgegengesetzte Kraft auf das medizinische Element ausgeübt, so wird dieses bis zum Erreichen eines bestimmen Betrages der Kraft aufgrund der Formschlußwirkung des in die Vertiefung eingreifenden Vorsprungs in seiner eingesetzten Position zurückgehalten. Überschreitet die Krafteinen bestimmten Betrag, so wird die Verbindung zwischen dem medizinischen Element und dem Implantat wieder aufgehoben, wobei das elastische Clipelement einschließlich seines Vorsprungs um einen gewissen Betrag radial nach innen verlagert werden und dabei aus der Hinterschneidung der Vertiefung in der inneren Mantelfläche der Aufnahmeausnehmung vortreten. Sobald der Vorsprung vollständig aus der Vertiefung hervorgetreten ist, läßt sich das Element ohne größeren Kraftaufwand weiter in axiale Richtung aus der Aufnahmeausnehmung entfernen.

Das erfindungsgemäße Implantat zeichnet sich somit durch eine Verbindungsart zu einem darin einsetzbaren medizinischen Element aus; die eine sehr einfache und schnelle Herstellung sowie Auflösung der Verbindung erlaubt. Dabei ist der Betrag einer der Einsetzbewegung entgegengesetzten Kraft, durch die sich die Verbindung wieder aufheben läßt, durch geeignete Auswahl der Geometrie- und Materialparameter beliebig einstellbar. Bei dem Implantat gemäß der Erfindung läßt sich ein medizinisches Element daher in kürzester Zeit einsetzen und wieder entfernen, wodurch sich - insbesondere bei einer Vielzahl gleichzeitig bearbeiteter Implantateder Zeitaufwand bei einer Behandlung und somit auch die Behandlungskosten nicht unwesentlich reduzieren lassen.

Gemäß einer vorteilhaften Ausgestaltung der Erfindung sind mehrere Clipelemente an dem distalen Ende des Verbindungszapfens angeordnet. Auf diese Weise läßt sich die radiale Elastizität der Clipelemente am einfachsten herstellen, wobei die Mehrzahl der Clipelemente eine besondere sichere Fixierung des medizinischen Elements bewirkt.
Auch wenn an dem medizinischen Element eine Mehrzahl von Clipelementen vorhanden ist, läßt sich der Fertigungsaufwand für die Vertiefung in dem Implantat gering halten, wenn die innere Mantelfläche der Aufnahmeausnehmung mit einer Ringnut versehen ist, in die die Vorsprünge der Clipelemente eingreifen.

Ist die innere Mantelfläche mit mehreren im Abstand und parallel zueinander angeordneten Ringnuten versehen, so läßt sich das medizinische Element entweder in verschiedenen Stellungen im Implantat fixieren, oder aber es ist möglich, medizinische Elemente mit unterschiedlich weit von einem Kopfbereich entfernten Clipelementen in ein und dasselbe Implantat einzusetzen.

Die Erfindung weiter ausgestaltend ist vorgesehen, daß die Clipelemente einstückig mit dem Verbindungszapfen ausgebildet sind, von dem ein Oberteil des medizinischen Elements entnehmbar ist.

Um die Auflösung der Verbindung zwischen dem Implantat und dem medizinischen Element auf einfache Weise ermöglichen zu können, wird vorgeschlagen, daß das medizinische Element an einem Oberteil, das sich in einem mit dem Implantat verbundenen Zustand des medizinischen Elements außerhalb des Implantats befindet, mit mindestens einer Vertiefung versehen ist, in die angepaßte Greifelemente eines Werkzeugs einbringbar sind.

Eine besonders einfache Ausgestaltung einer solchen Vertiefung besteht darin, daß das medizinische Element an seinem Oberteil eine in dessen äußerer Mantelfläche umlaufende Ringnut aufweist. Weist diese Ringnut einen V-förmigen Querschnitt auf, lassen sich darin beispielsweise angepaßt V-förmige Backen einer Zange einführen, mit der das medizinische Element aus dem Implantat herausgezogen werden kann.

Eine besonders vorteilhafte Verwendung des erfindungsgemäßen Implantats besteht darin, daß es in einen Kieferknochen einbringbar ist und daß das medizinische Element eine Verschlußkappe, ein Gingiva-Former, ein Abdruckpfosten oder ein Ersatzzahn ist.

Das erfindungsgemäße Implantat wird nachfolgend anhand eines Ausführungsbeispiels, das in der Zeichnung dargestellt ist, näher erläutert.

Es zeigt:
- Figur 1: ein Implantat mit einer eingeclipsten Verschlußkappe im Längsschnitt;
- Figur 2: eine Draufsicht auf die Verschlußkappe;
- Figur 3: eine Draufsicht auf das Implantat nach Entfernung der Verschlußkappe;
- Figur 4: einen Querschnitt entlang der Linie IV-IV durch das Implantat gemäß Figur 1;
- Figur 5: einen Querschnitt entlang der Linie V-V durch das Implantat gemäß Figur 1;
- Figur 6: einen vergrößerten Ausschnitt des Eingriffsbereichs eines Clipelements;
- Figur 7: wie Figur 1, jedoch mit aufgeclipstem Gingiva-Former;
- Figur 8: wie Figur 7, jedoch mit Schlitz im Gingiva-Former für transgingivale Einheilung;
- Figur 9: einen Rohling für einen provisorischen Ersatzzahn in einem Laborimplantat;
- Figur 10: wie Figur 9, jedoch eines fertigen provisorischen Ersatzzahns und
- Figur 11: wie Figur 10, jedoch im erfindungsgemäßen Implantat.

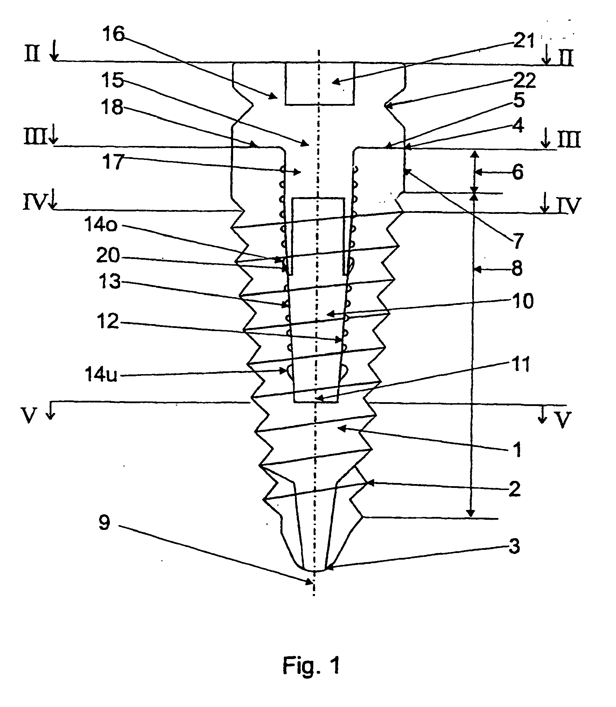

Den Figuren 1 bis 5 läßt sich ein aus Titan bestehendes Implantat 1 entnehmen, das eine ungefähr konische äußere Grundform besitzt und an seiner äußeren Mantelfläche mit einem Außengewinde 2 versehen ist. Das Implantat 1 besitzt ein abgerundetes distales Ende 3 und ein proximales Ende 4, das von einer im wesentlichen kreisringförmigen Kontaktfläche 5 gebildet wird. In einem an die Kontaktfläche 5 anschließenden Abschnitt 6 besitzt das Implantat 1 eine zylindrische Form mit einer hochglanzpolierten Mantelfläche 7. In einem darauf folgenden Gewindeabschnitt 8 ist das Implantat 1 konisch ausgeformt. Ausgehend von der Kontaktfläche 5 erstreckt sich parallel zu einer Längsachse 9 des Implantats 1 eine Aufnahmeausnehmung 10, die über die gesamte Länge des Abschnitts 6 sowie einen Teil der Länge des Gewindeabschnitts 8 verläuft.

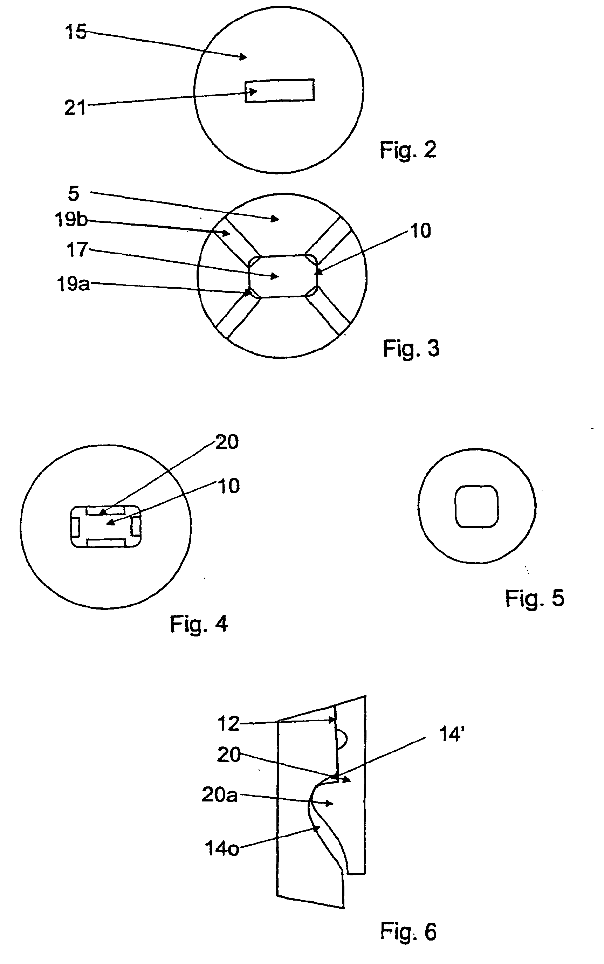

Wie sich der Figur 3 entnehmen läßt besitzt der Querschnitt der Aufnahmeausnehmung 10 im Bereich des Abschnitts 6 durchgängig die Form eines abgerundeten Rechtecks. Beginnend mit dem Gewindeabschnitt 8 verjüngt sich der Querschnitt der Aufnahmeausnehmung 10 dahingehend kontinuierlich, daß am Grund 11 der Aufnahmeausnehmung 10 der Querschnitt die Form eines abgerundeten Quadrats (vgl. Figur 5) aufweist. Der Übergang von der abgerundeten rechteckigen zur abgerundeten quadratischen Querschnittsform erfolgt kontinuierlich und ohne Sprünge.

Wie sich insbesondere der Figur 1 entnehmen läßt, ist die Wandung 12 der Aufnahmeausnehmung 10 mit einer Vielzahl von Ringnuten 13 versehen, die senkrecht zur Längsachse 9 ausgerichtet sind. Des weiteren ist die Wandung 12 mit einer oberen und einer unteren Ringnut 14o und 14u versehen, deren Funktion später erläutert wird.

In das in Figur 1 dargestellte Implantat ist eine Verschlußkappe 15 eingesetzt, die aus einem ungefähr zylinderförmigem Kopfteil 16 und einem koaxial hierzu ausgerichteten Verbindungszapfen 17 besteht, der sich in die Aufnahmeausnehmung 10 erstreckt. Eine Kontaktfläche 18 des Kopfteils 16 kommt an der Kontaktfläche 5 des Implantats 1 kraftschlüssig zur Anlage.

Wie sich der Figur 3 entnehmen läßt, weist der Verbindungszapfen 17 in einem oberen Abschnitt einen ungefähr rechteckförmigen Querschnitt auf, wobei die Eckbereiche derart gebrochen sind, daß in den Rundungsbereich des Querschnitts der Aufnahmeausnehmung 10 zwischen dem Verbindungszapfen 17 und der Wandung 12 der Aufnahmeausnehmung 10 vier Entlüftungskanäle 19a gebildet werden. Beim Einschieben des Verbindungszapfens 17 in die Aufnahmeausnehmung 10 verdrängte Luft kann daher, ohne daß es zu einem Demontagevorgang behinderten Druckaufbau kommt, nach oben abgeführt werden, wobei sich die Luft durch vier radial nach außen verlaufende Entlüftungsnuten 19b, die in die Stirnfläche 5 des Implantats 1 eingebracht sind und mit den Entlüftungskanälen 19a kommunizieren, nach außen entweichen kann.

Im Einbauzustand liegen die äußeren Mantelflächen des Verbindungszapfens 17 an der Wandung 12 der Aufnahmeausnehmung 10 sowie die beiden Kontaktflächen 5 und 18 kraftschlüssig aneinander.

Da die Verschlußkappe 15 lediglich temporär nach der Implantation an dem Implantat 1 verbleibt, ist sie lediglich mit Hilfe von 4 Clipelementen 20, die in die obere Ringnut 140 eingreifen, mit dem Implantat verbunden.
Wie sich insbesondere aus Figur 6 erkennen läßt, weisen die stabförmigen Clipelemente 20 in der Nähe ihres distalen Endes einen radial nach außen vorstehenden Vorsprung 20a auf der vollständig innerhalb der Ringnut 140 angeordnet ist. Bis zu dem distalen Ende des elastischen Clipelements 20 hin verjüngt sich der Vorsprung 20a zunehmend, so daß die Verschlußkappe ohne weiteres in die Aufnahmeausnehmung 10 eingeführt werden kann.

Der mit Hilfe der Clipelemente 20 hergestellte Formschluß zwischen dem Implantat 1 und der Verschlußkappe 15 bzw. einem beliebigen anderen medizinischen Element ist dadurch wieder aufhebbar, daß die Verschlußkappe 15, ausgehend von dem in Figur 1 dargestellten Einbauzustand, axial nach oben herausgezogen wird. Wenn eine nach oben gerichtete Axialkraft auf die Verschlußkappe 15 ausgeübt wird, wird diese zunächst aufgrund der formschlüssigen Verbindung in der Einbauposition gehalten. Übersteigt die Kraft jedoch einen bestimmten Betrag, so werden die Clipelemente 20 radial nach innen ausgelenkt, weil die Vorsprünge 20a auf dem oberen Abschnitt der Wandung der Ringnut 140 mit einer Komponente senkrecht zu der Längsachse 11 des Implantats 1 geführt werden. Sobald der am weitesten radial nach außen vorstehende Teil des Vorsprungs 20a die obere Kante 14' der Ringnut 140 erreicht hat, ist der Formschluß aufgehoben und die Verbindungskappe 15 läßt sich mit geringerer Kraft vollständig aus der Aufnahmeausnehmung 10 des Implantats 1 herausziehen.

Anstelle des in Figur 1 gezeigten Eingriffs der Clipelemente 20 in die obere Ringnut 14o ist bei einem entsprechend verlängerten Verbindungszapfen 17 bzw. bei verlängerten Clipelementen 20 auch ein Eingriff in die untere Ringnut 14u möglich.

Die Verschlußkappe 15 wird bereits vom Hersteller des Implantats 1 in dieses eingesetzt und dient einerseits dazu, das Implantat 1 nach Anfertigung einer entsprechenden Bohrung im Knochen mit Hilfe eines Schraubendrehers, der in den in Figur 2 gezeigten Schlitz 21 eingreift, einzudrehen. Aufgrund des annähernd rechteckförmigen Querschnitts des Verbindungszapfens 17 und der angepaßten Aufnahmeausnehmung 10 ist eine Drehmomenteinleitung über die Verschlußkappe 15 in das Implantat 1 möglich. Nach der Implantation verbleibt die Verschlußkappe 15 am Implantat 1, um zum anderen die Aufnahmeausnehmung 10 vor äußeren Verschmutzungen zu schützen.

Ca. drei bis sechs Monate nach Einsetzen des Implantats in den Kieferknochen ist die Einheilung so weit abgeschlossen, daß die die Verschlußkappe 15 abdeckende Schleimhaut in einer zweiten Operation wieder geöffnet werden kann. Die Verschlußkappe 15 wird entfernt, wozu mit Hilfe eines zangenartigen Werkzeugs in eine V-förmige Ringnut 22 in dem Kopfteil 16 eingegriffen wird und hierdurch die gesamte Verschlußkappe 15 durch einen leichten Ruck in axiale Richtung nach oben aus dem Implantat 1 entfernt wird. Der durch die Clipelemente 20 hervorgerufene Formschluß wird hierbei unter Ausnutzung der Elastizität der Clipelemente 20 aufgehoben.

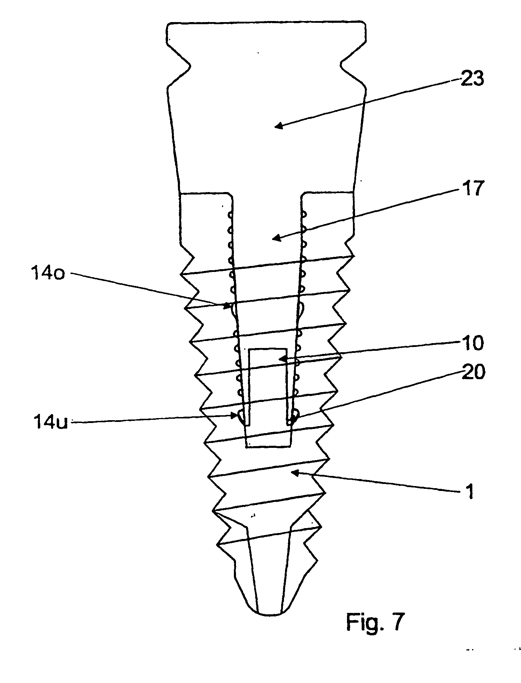

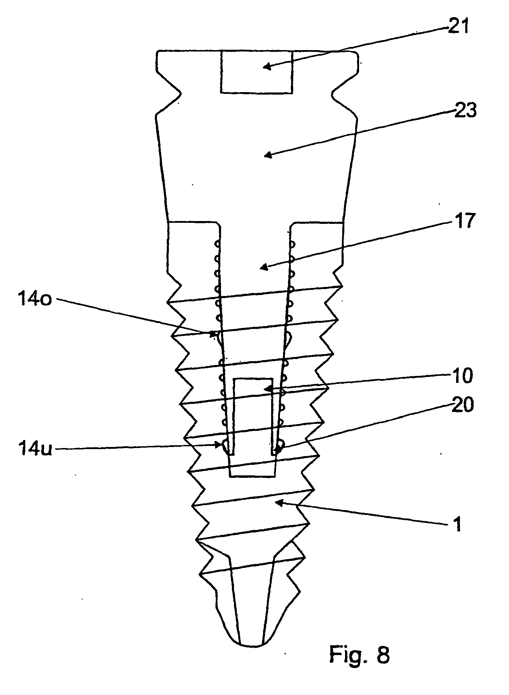

In die Aufnahmeausnehmung 10 wird nunmehr ein Verbindungszapfen 17 eines Gingiva-Formers 23 eingesetzt, wie er in Figur 7 dargestellt ist. Das Befestigungsprinzip ist dasselbe wie bei der Verschlußkappe 15. Exemplarisch ist in Figur 8 dargestellt, daß die Clipelemente 20 des Gingiva-Formers 23 in die untere Ringnut 14u einrasten. Ebenso ist jedoch ein Ginigiva-Former 23 denkbar, bei dem die Clipelemente 20 in die obere Ringnut 140 eingreifen.

Figur 8 zeigt einen Gingiva-Former mit Schlitz, wie er bei transgingivaler Einheilung des Implantats Verwendung findet. In diesem Fall kommt keine Verschlußkappe zur Anwendung, sondern das Implantat wird direkt zusammen mit dem Gingiva-Former in den Kieferknochen eingeschraubt.

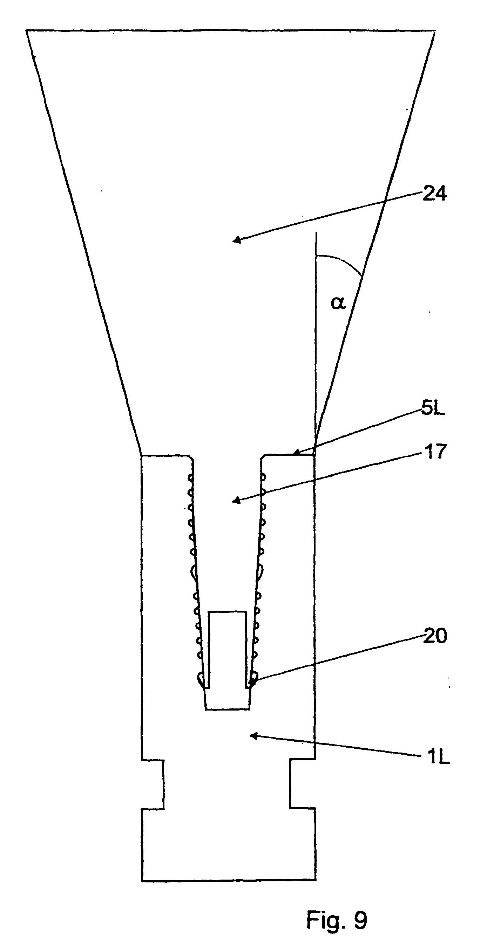

Figur 9 zeigt ein Laborimplantat 1L, dessen Aufnahmeausnehmung mit der des Implantats 1 übereinstimmt und in das ein Rohling 24 eines provisorischen Ersatzzahns, ebenfalls mit Hilfe eines Verbindungszapfens 17, eingesetzt ist. Der Rohling 24 ist kegelstumpfförmig und erweitert sich ausgehend von der Stirnfläche 5L des Laborimplantats 1 L unter einem Winkel α von 15°. Auf diese Weise lassen sich Schiefstellungen des Implantats 1 bzw. 1L gegenüber den Nachbarzähnen oder weiteren Implantaten in weiten Winkelbereichen in alle Richtungen ausgleichen. Der Verbindungszapfen 17 des Rohlings 24 ist ebenfalls mit Clipelementen 20 versehen, die ein unkompliziertes Fixieren und Entnehmen des Rohlings 24 gewährleisten.

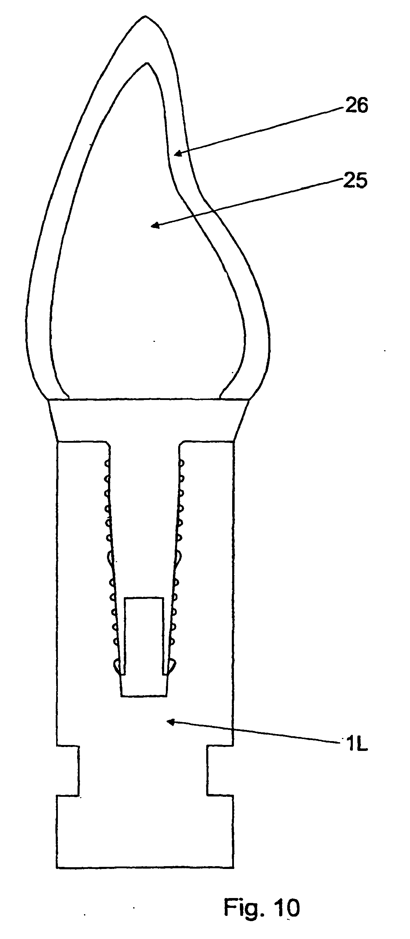

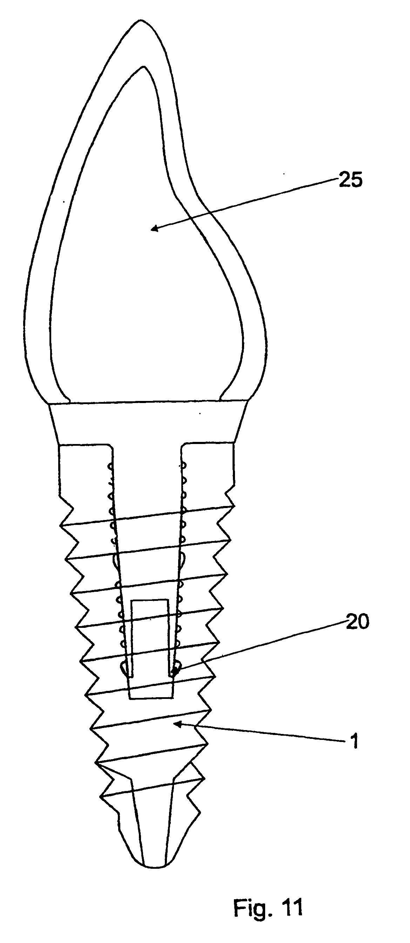

Figur 10 zeigt einen fertig bearbeiteten provisorischen Ersatzzahn 25, der durch spanende Bearbeitung des Rohlings 24 in einem zahntechnischen Labor gefertigt wurde. Auf den geschliffenen Rohling ist eine äußere Keramikschicht 26 aufgebrannt. Der fertige provisorische Ersatzzahn 25 kann anschließend beim Patienten in das Implantat 1 eingeführt (Figur 11) und dort mit Hilfe der Clipelemente 20 solange fixiert werden, bis der Patient mit dem endgültigen Ersatzzahn 28 versorgt werden kann.

## Patentansprüche

1. Einrichtung mit einem medizinischen Element und einem Implantat (1) zur Aufnahme eines Verbindungszapfens (17) des medizinischen
Elements, wobei das Implantat mit einer Längsachse (9), einem distalen Ende (3) und einem proximalen Ende (4), von dem aus sich eine Aufnahmeausnehmung (10) für den Verbindungszapfen (17) in Richtung der Längsachse (9) in das Innere des Implantats (1) erstreckt versehen ist, wobei das Implantat (1) an seiner äußeren Mantelfläche kraft- oder formschlüssig mit der inneren Mantelfläche einer Aufnahmebohrung in einem Knochen eines menschlichen oder tierischen Körpers verbindbar ist und wobei der an die Aufnahmeausnehmung (10) angepaßte Verbindungszapfen (17) kraft- oder formschlüssig mit der inneren Mantelfläche der Aufnahmeausnehmung (10) verbindbar ist, wobei die innere Mantelfläche der Aufnahmeausnehmung (10) mit mindestens einer senkrecht zur Längsachse (9) des Implantats (1) ausgerichteten und eine Hinterschneidung bildenden Vertiefung versehen ist, mit der ein radial nach außen vorstehender Vorsprung (20a) eines elastischen Clipelements (20) des Verbindungszapfens (17) in Eingriff bringbar ist, **dadurch gekennzeichnet, dass** der Querschnitt der Aufnahmeausnehmung (10) im Bereich des proximalen des Implantats Abschnitts (6) durchgängig die Form eines abgerundeten Rechtecks besitzt und sich, beginnend mit dem daraüf folgenden Gewindeabschnitt (8), kontinuierlich verjüngt und am Grund (11) der Aufnahmeausnehmung (10) die Form eines abgerundeten Quadrats aufweist und dass der Verbindungszapfen (17) in einem oberen Abschnitt einen ungefähr rechteckförmigen Querschnitt aufweist.

2. Einrichtung nach Anspruch 1, **dadurch gekennzeichnet, daß** mehrere Clipelemente (20) an dem distalen Ende des Verbindungszapfens (17) angeordnet sind.

3. Einrichtung nach Anspruch 1 oder 2, **dadurch gekennzeichnet, daß** die innere Mantelfläche der Aufnahmeausnehmung (10) mit einer Ringnut (140, 14u) versehen ist, in die die Vorsprünge (20a) der Clipelemente (20) eingreifen.

4. Einrichtung nach Anspruch 3, **dadurch gekennzeichnet, daß** die innere Mantelfläche mit mehreren im Abstand und parallel zueinander angeordneten Ringnuten (14o, 14u) versehen ist.

5. Einrichtung nach einem der Ansprüche 1 bis 4, **dadurch gekennzeichnet, daß** die Clipelemente (20) einstückig mit dem Verbindungszapfen (17) ausgebildet sind und von diesem ein Oberteil (16) des medizinischen Elements entfernbar ist.

6. Einrichtung nach einem der Ansprüche 1 bis 5, **dadurch gekennzeichnet, daß** das medizinische Element an einem Oberteil (16), das sich in einem mit dem Implantat (1) verbundenen Zustand des medizinischen Elements außerhalb des Implantats (1) befindet, mit mindestens einer Vertiefung versehen ist, in die angepaßte Greifelemente eines Werkzeugs einbringbar sind.

7. Einrichtung nach Anspruch 6, **dadurch gekennzeichnet, daß** das medizinische Element an seinem Oberteil (16) eine in dessen äußerer Mantelfläche umlaufende Ringnut (22) aufweist.

8. Einrichtung nach Anspruch 7, **dadurch gekennzeichnet, daß** die Ringnut (22) einen V-förmigen Querschnitt besitzt.

9. Einrichtung nach einem der Ansprüche 1 bis 8, **dadurch gekennzeichnet, daß** es in einen Kieferknochen einbringbar ist und daß das medizinische Element eine Verschlußkappe (15), ein Gingiva-Former (23), ein Abdruckpfosten oder ein Ersatzzahn (25) ist.

## Claims

1. Device with a medical element and an implant (1) for receiving a connecting pin (17) of the medical element, wherein the implant is provided with a longitudinal axis (9), a distal end (3) and a proximal end (4), from which a receiving recess (10) for the connecting pin (17) extends in the direction of the longitudinal axis (9) into the interior of the implant (1), wherein the implant (1), at its outer generated surface, can be connected non-positively or positively to the inner generated surface of a receiving bore in a bone of a human or animal body and wherein the connecting pin (17) adapted to the receiving recess (10) can be non-positively or positively connected to the inner generated surface of the receiving recess (10), the inner generated surface of the receiving recess (10) being provided with at least one indentation aligned perpendicularly to the longitudinal axis (9) of the implant (1) and forming an undercut, with which a projection (20a) projecting radially outwardly of an elastic clip element (20) of the connecting pin (17) can be engaged, **characterised in that** the cross section of the receiving recess (10) in the region of the proximal portion (6) of the implant continuously has the form of a rounded rectangle and tapers continuously, beginning with the subsequent threaded portion (8) and, at the base (11) of the receiving recess (10), has the form of a rounded square and **in that** the connecting pin (17) in an upper portion has an approximately rectangular cross section.

2. Device according to claim 1, **characterised in that** a plurality of clip elements (20) are arranged at the distal end of the connecting pin (17).

3. Device according to claim 1 or 2, **characterised in that** the inner generated surface of the receiving recess (10) is provided with an annular groove (140, 14u), in which the projections (20a) of the clip elements (20) engage.

4. Device according to claim 3, **characterised in that** the inner generated surface is provided with a plurality of annular grooves (140, 14u) arranged at a spacing from one another and parallel to one another.

5. Device according to any one of claims 1 to 4, **characterised in that** the clip elements (20) are configured integrally with the connecting pin (17) and an upper part (16) of the medical element can be removed therefrom.

6. Device according to any one of claims 1 to 5, **characterised in that** the medical element on an upper part (16), which, in a state of the medical element connected to the implant (1), is located outside the implant (1), is provided with at least one indentation, into which adapted gripper elements of a tool can be introduced.

7. Device according to claim 6, **characterised in that** the medical element at its upper part (16) has a peripheral annular groove (22) in its outer generated surface.

8. Device according to claim 7, **characterised in that** the annular groove (22) has a V-shaped cross section.

9. Device according to any one of claims 1 to 8, **characterised in that** it can be introduced into a jawbone and **in that** the medical element is a closure cap (15), a gum shaper (23), an impression post or a false tooth (25).

## Revendications

1. Système comprenant un élément médical et un implant (1) destiné à recevoir un tenon de raccordement (17) de l'élément médical,
dans lequel l'implant est pourvu d'un axe longitudinal (9), d'une extrémité distale (3) et d'une extrémité proximale (4) à partir de laquelle s'étend, à l'intérieur de l'implant (1), dans la direction de l'axe longitudinal (9), un évidement de réception (10) pour le tenon de raccordement (17),
dans lequel l'implant (1) peut être relié, au niveau de sa surface périphérique extérieure, par adhérence ou complémentarité de forme, avec la surface périphérique intérieure d'un alésage de réception dans un os d'un corps humain ou animal, et
dans lequel le tenon de raccordement (17) adapté à l'évidement de réception (10) peut être relié par adhérence ou complémentarité de forme à la surface périphérique intérieure de l'évidement de réception (10),
et dans lequel la surface périphérique intérieure de l'évidement de réception (10) est munie d'au moins un creux orienté perpendiculairement à l'axe longitudinal (9) de l'implant (1) et formant une contre-dépouille, avec laquelle peut être amenée en prise une protubérance (20a) en saillie radiale vers l'extérieur, d'un élément d'encliquetage (20) du tenon de raccordement (17),
**caractérisé en ce que** la section transversale de l'évidement de réception (10) possède, dans la zone du tronçon proximal (6) de l'implant, une forme continue de rectangle arrondi, et se rétrécit de manière continue à partir du tronçon fileté (8) succédant au précédent, et présente au niveau du fond (11) de l'évidement de réception (10), la forme d'un carré arrondi, et **en ce que** le tenon de raccordement (17) présente, dans un tronçon supérieur, une section transversale approximativement de forme rectangulaire.

2. Système selon la revendication 1, **caractérisé en ce que** plusieurs éléments d'encliquetage (20) sont agencés à l'extrémité distale du tenon de raccordement (17).

3. Système selon la revendication 1 ou 2, **caractérisé en ce que** la surface périphérique intérieure de l'évidement de réception (10) est pourvue d'une rainure annulaire (14o, 14u) dans laquelle s'engagent les protubérances (20a) des éléments d'encliquetage (20).

4. Système selon la revendication 3, **caractérisé en ce que** la surface périphérique intérieure est pourvue de plusieurs rainures annulaires (14o, 14u) agencées à distance les unes des autres et parallèlement les unes aux autres.

5. Système selon l'une des revendications 1 à 4, **caractérisé en ce que** les éléments d'encliquetage (20) sont réalisés d'un seul tenant avec le tenon de raccordement (17), et une partie supérieure (16) de l'élément médical peut être retirée de celui-ci.

6. Système selon l'une des revendications 1 à 5, **caractérisé en ce que** l'élément médical est pourvu, au niveau d'une partie supérieure (16), qui, dans un état de liaison de l'élément médical avec l'implant (1), se trouve à l'extérieur de l'implant (1), d'au moins un creux dans lequel ou lesquels peuvent être engagés des éléments de préhension adaptés d'un outil.

7. Système selon la revendication 6, **caractérisé en ce que** l'élément médical présente, sur sa partie supérieure (16), dans sa surface périphérique extérieure, une rainure annulaire (22) périphérique.

8. Système selon la revendication 7, **caractérisé en ce que** la rainure annulaire (22) possède une section transversale en forme de V.

9. Système selon l'une des revendications 1 à 8, **caractérisé en ce qu'**il peut être implanté dans un os de mâchoire, et **en ce que** l'élément médical est un cabochon de fermeture (15), un élément de mise en forme ou de contention de gencive (23), un pivot de prise d'empreinte ou une prothèse de dent (25).
